(19) Europäisches Patentamt
European Patent Office
Office européen des brevets

(11) Veröffentlichungsnummer: **0 012 366**
**B1**

(12) # EUROPÄISCHE PATENTSCHRIFT

(45) Veröffentlichungstag der Patentschrift: **14.03.84**

(21) Anmeldenummer: **79104956.2**

(22) Anmeldetag: **06.12.79**

(51) Int. Cl.³: **C 07 D 237/26,** A 61 K 31/50 // C07C62/38, C07C79/44, C07C97/10, C07C101/78, C07C103/46, C07C121/76

(54) **Neue 3,4-Diaza-bicyclo(4.1.0)hepten-(2)-one-(5), Verfahren zu ihrer Herstellung und diese Verbindungen enthaltende therapeutische Mittel.**

(30) Priorität: **16.12.78 DE 2854475**

(43) Veröffentlichungstag der Anmeldung: **25.06.80 Patentblatt 80/13**

(45) Bekanntmachung des Hinweises auf die Patenterteilung: **14.03.84 Patentblatt 84/11**

(84) Benannte Vertragsstaaten: **AT BE CH DE FR GB IT LU NL**

(56) Entgegenhaltungen:
**EP - A - 0 000 113**
**FR - A - 2 110 329**

**CHIMIE THERAPEUTIQUE, Band 6, Nr. 2, März-April 1971, C.G. WERMUTH: "Dérivés pyridaziniques présentant un intérêt thérapeutique. VII. Synthèses d'analoques de la morpholino éthyl-2 méthyl-4 phényl-6 pyridazone-3 modifies au niveau des atomes de carbone *4 et 5*"; *Seiten 109-115***

**Die Akte enthält technische Angaben, die nach dem Eingang der Anmeldung eingereicht wurden und die nicht in dieser Patentschrift enthalten sind.**

(73) Patentinhaber: **BASF Aktiengesellschaft, Carl-Bosch-Strasse 38, D-6700 Ludwigshafen (DE)**

(72) Erfinder: **Thyes, Marco, Dr. Dipl.-Chem., Mundenheimer Strasse 148, D-6700 Ludwigshafen (DE)**
Erfinder: **Gries, Josef, Dr., Roemerweg 43, D-6706 Wachenheim (DE)**
Erfinder: **Lehmann, Hans Dieter, Dr., Im Hefen 15, D-6945 Hirschberg-Leutershausen (DE)**
Erfinder: **Lenke, Dieter, Dr., Kekuleplatz 1, D-6700 Ludwigshafen (DE)**
Erfinder: **Kunze, Johannes, Dr., Pfarrweg 5, D-8343 Triftern (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



BUNDESDRUCKEREI BERLIN

# Neue 3,4-Diaza-bicyclo[4.1.0]hepten-(2)-one-(5), Verfahren zu ihrer Herstellung und diese Verbindungen enthaltende therapeutische Mittel

Die Erfindung betrifft neue 2-Aryl-3,4-diaza-bicyclo[4.1.0]hepten-(2)-one-(5), Verfahren zu ihrer Herstellung, diese Verbindungen enthaltende pharmazeutische Zubereitungen und ihre Verwendung bei der Prophylaxe und Therapie thrombo-embolischer Erkrankungen und bei hohem Blutdruck.

In der deutschen Patentanmeldung P 2 727 481.3 werden 6-(p-Alkanoylaminophenyl)-4,5-dihydro-3(2 H)-pyridazinone, die im Pyridazinonring in 5-Stellung gegebenenfalls einen Alkylrest aufweisen und im Alkanoylrest durch ein Halogenatom substituiert sind, bei der Behandlung von thrombo-embolischen Erkrankungen und zu hohen Blutdrucks vorgeschlagen. Für andere 6-Aryl-4,5-dihydro-3(2 H)-pyridazinone werden beispielsweise in den DE-OS 2 150 436 und 2 207 517 blutdrucksenkende Eigenschaften beschrieben.

Das 2-Phenyl-3,4-diaza-bicyclo[4.1.0]hepten-(2)-on-(5) wird beispielsweise von G. Maier in Chem. Ber. 98, 2438–2445 (1965) beschrieben. Über pharmakologische Wirkungen dieser Verbindung ist nichts bekannt. Weiterhin ist das am 4-ständigen N-Atom durch eine Morpholinoethylgruppe substituierte 2-Phenyl-3,4-diaza-bicyclo[4.1.0]hepten-(2)-on-(5) bekannt (Chim. Thér. 1971, 6, 109–115), für das analgetische und sedative Eigenschaften angegeben werden. Aus der US-PS 3 931 176 ist bekannt, daß am 4-ständigen Stickstoffatom substituierte 2-Aryl-3,4-diaza-bicyclo[4.n.0]en-(2)-one-(5) (n = 2, 3 oder 4) zentraldämpfende Eigenschaften besitzen.

Es wurde nun gefunden, daß Diaza-bicyclo[4.1.0]heptone der allgemeinen Formel I,

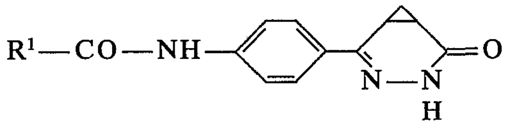

(I)

in der $R^1$ für ein Wasserstoffatom, einen gegebenenfalls durch 1 bis 3 Halogenatome substituierten Alkylrest mit 1 bis 4 C-Atomen, einen gegebenenfalls ein- bis dreifach durch Halogenatome und/oder Methylreste substituierten Cycloalkylrest mit 3 bis 5 C-Atomen im Ring oder einen Alkenylrest mit 2 bis 4 C-Atomen steht, wertvolle pharmakologische Eigenschaften aufweisen.

Als Alkylreste mit 1 bis 4 C-Atomen für den Rest $R^1$, geradkettig oder verzweigt, sind beispielsweise Methyl, Ethyl, Propyl, Isopropyl, Butyl, Isobutyl, sek.-Butyl und tert.-Butyl zu nennen.

Durch Halogen, wie Chlor, Brom, Fluor, Jod, substituierte Alkylreste mit 1 bis 4 C-Atomen für den Rest $R^1$, geradkettig oder verzweigt, sind beispielsweise Chlormethyl, Brommethyl, Fluormethyl, Jodmethyl, 1-Chlorethyl, 1-Bromethyl, 1-Fluorethyl, 1-Jodethyl, 2-Chlorethyl, 2-Bromethyl, 2-Fluorethyl, 2-Jodethyl, 1-Chlorpropyl, 1-Brompropyl, 1-Fluorpropyl, 1-Jodpropyl, 2-Chlorpropyl, 2-Brompropyl, 3-Chlorpropyl, 3-Brompropyl, 3-Fluorpropyl, 1-Chlorisopropyl, 1-Bromisopropyl, 1-Jodisopropyl, 2-Chlorisopropyl, 2-Bromisopropyl, 1-Chlorbutyl, 1-Brombutyl, 1-Fluorbutyl, 4-Chlorbutyl, 4-Brombutyl, 1-Chlorisobutyl, 1-Bromisobutyl, 2-Chlorisobutyl, 1-Chlor-sek.-butyl, 1-Brom-sek.-butyl, 3-Chlor-sek.-butyl, Chlor-tert.-butyl, Brom-tert.-butyl, Dichlormethyl, Difluormethyl, 1,1-Dichlorethyl, 1,2-Dichlorethyl, 1,2-Dibromethyl, 2,2-Dichlorethyl, 1,1-Dichlorpropyl, 1,2-Dichlorpropyl, 1,2-Dibrompropyl, 1,3-Dichlorpropyl, 2,3-Dibrompropyl, 1,2-Dichlorisopropyl, 1,4-Dichlorbutyl, 1,2-Dibromisobutyl, 1,1-Bis-chlormethyl-ethyl, Trichlormethyl, Trifluormethyl, Chlordifluormethyl, 1,1,2,2-Tetrafluorethyl.

Die Alkylreste $R^1$ sind gegebenenfalls vorzugsweise durch Fluor, Chlor oder Brom substituiert.

Als Cycloalkylreste mit 3 bis 5 C-Atomen im Ring für den Rest $R^1$, unsubstituiert oder substituiert, sind beispielsweise Cyclopropyl, 1-Methylcyclopropyl, 2-Methylcyclopropyl, 2,2-Dimethylcyclopropyl, 1-Chlorcyclopropyl, 2-Bromcyclopropyl, 2,2-Dichlorcyclopropyl, 2,2-Dibromcyclopropyl, 2,2-Dichlor-1-methylcyclopropyl, Cyclobutyl, 1-Methylcyclobutyl, 2-Methylcyclobutyl, 3-Methylcyclobutyl, 3,3-Dimethylcyclobutyl, 1-Chlorcyclobutyl, 2-Chlorcyclobutyl, 3-Chlorcyclobutyl, 1-Bromcyclobutyl, 1-Brom-3,3-dimethylcyclobutyl, Cyclopentyl, 1-Methylcyclopentyl, 2,5-Dimethylcyclopentyl, 1-Chlorcyclopentyl und 3,4-Dichlorcyclopentyl zu nennen.

Alkenylreste mit 2 bis 4 C-Atomen für $R^1$ sind beispielsweise Vinyl, Propenyl, Isopropenyl, Allyl, But-1-enyl, But-2-enyl, But-3-enyl, 2-Methyl-prop-1-enyl.

Die Verbindungen der Formel I werden durch Cyclisierung einer cis-2-(p-Acylaminobenzoyl)-cyclopropancarbonsäure der Formel III, in der $R^1$ die für den gleichen Rest der Formel I angegebenen Bedeutungen hat, mit Hydrazin in an sich üblicher Weise erhalten.

Die Ausgangsverbindungen der Formel III werden durch Umsetzung eines Anilids der Formel II, in der $R^1$ die für den gleichen Rest der Formel I angegebenen Bedeutungen hat, mit cis-Cyclopropan-1,2-dicarbonsäureanhydrid in Gegenwart von Aluminiumchlorid unter den Bedingungen einer Friedel-Crafts-Acylierung erhalten.

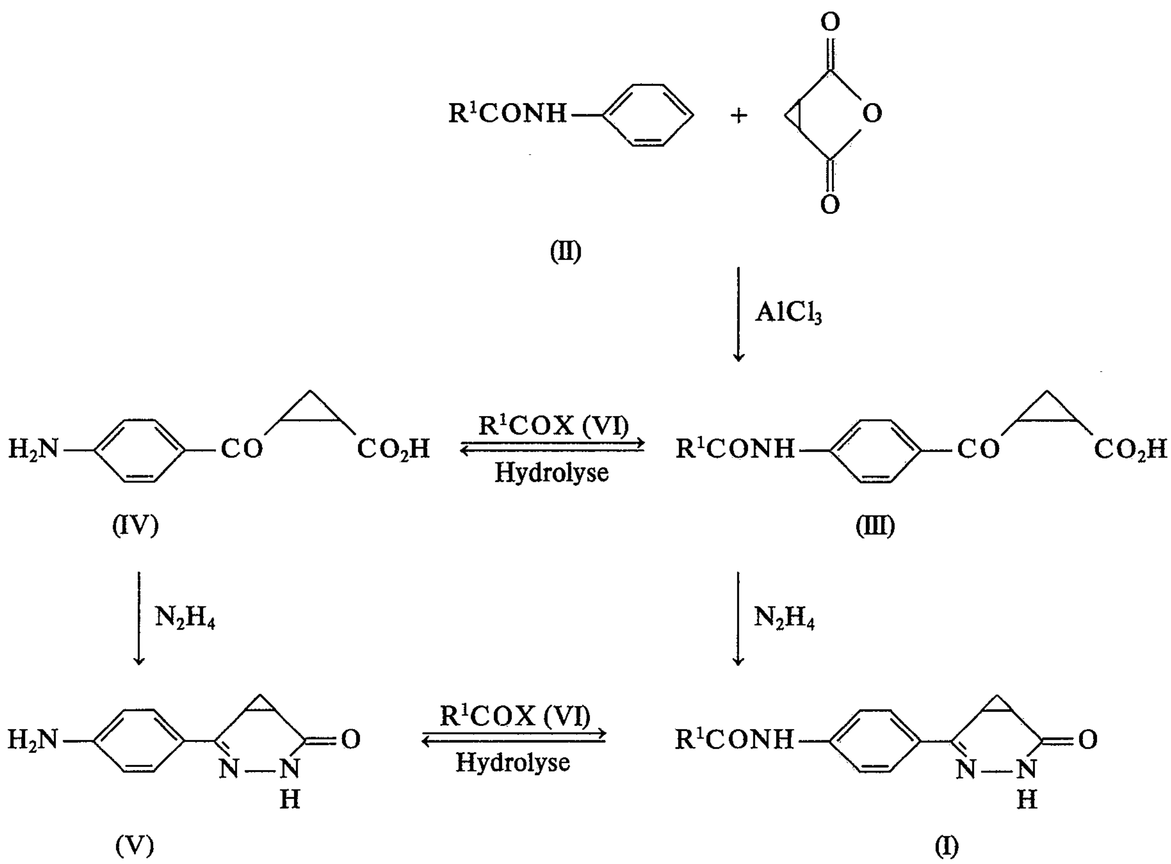


(II) (IV) (III) (V) (I)

Die Diaza-bicyclo[4.1.0]heptenone der Formel I lassen sich auch herstellen, indem man die Aminoverbindung V mit einem Acylierungsmittel der Formel VI,

$$R^1COX \qquad (VI)$$

in der $R^1$ die für den gleichen Rest der Formel I angegebenen Bedeutungen hat und X für ein Chloratom, OH, einen niederen Alkoxyrest oder einen Rest der Formel $OCOR^1$, in der $R^1$ die für Formel I angegebenen Bedeutungen hat, steht, umsetzt. Gemäß den für X angegebenen Bedeutungen sind zweckmäßige Acylierungsmittel die entsprechenden Carbonsäurechloride, Carbonsäuren, Carbonsäureester, insbesondere Methyl- und Ethylester, und die entsprechenden Carbonsäureanhydride.

Die Verbindung der Formel V läßt sich durch Hydrolyse eines Diaza-bicyclo[4.1.0]heptenons der Formel I, beispielsweise des 2-(p-Acetylaminophenyl)-3,4-diaza-bicyclo[4.1.0]hepten-(2)-ons-(5) ($R^1 = -CH_3$), herstellen. Diese Hydrolyse wird nach an sich bekannten Methoden, zum Beispiel mit wäßrigem Natriumhydroxid in Gegenwart eines niederen Alkohols, wie Methanol oder Ethanol, als Lösungsmittel bei Rückflußtemperatur durchgeführt.

Das Diaza-bicyclo[4.1.0]heptenon der Formel V kann auch erhalten werden, indem man die Aminosäure der Formel IV mit Hydrazin in üblicher Weise cyclisiert. Die Verbindung IV wird durch Hydrolyse einer cis-2-(p-Acylaminobenzoyl)-cyclopropancarbonsäure der Formel III, beispielsweise der cis-2-(p-Acetylaminobenzoyl)-cyclopropancarbonsäure ($R^1 = CH_3$), nach an sich üblichen Methoden, zum Beispiel mit wäßriger Salzsäure, erhalten.

Nach einem weiteren Herstellungsverfahren werden die Verbindungen der Formel I erhalten, indem man die Aminosäure der Formel IV mit einem Acylierungsmittel der Formel VI acyliert und die dabei gebildete Acylaminoverbindung der Formel III mit Hydrazin in üblicher Weise cyclisiert.

Die Verbindungen der Formel I, in denen $R^1$ einen durch Halogen substituierten Alkyl- bzw. Cycloalkylrest oder einen Alkenylrest bedeutet, werden bevorzugt über die Acylierung des Aminophenyl-diaza-bicyclo[4.1.0]heptenons der Formel V hergestellt.

Die Friedel-Crafts-Acylierung eines Anilids der Formel II mit cis-Cyclopropan-1,2-dicarbonsäureanhydrid zu einer Cyclopropancarbonsäure der Formel III kann in einem Lösungsmittel, beispielsweise Schwefelkohlenstoff, bei Temperaturen von 0 bis 60°C durchgeführt werden. Sie kann auch in einer Dimethylformamid/Aluminiumchlorid-Schmelze bei Temperaturen zwischen 50 und 120°C, vorzugsweise 60 bis 90°C, erfolgen. Dabei ist es zweckmäßig auf 1 Mol cis-Cyclopropan-1,2-dicarbonsäureanhydrid bzw. 1 Mol Anilid der Formel II etwa 10 Mol Aluminiumchlorid und etwa 2,5 Mol Dimethylformamid zu verwenden.

Die Acylierung des Diaza-bicyclo[4.1.0]heptenons V oder der Aminosäure IV mit einem Acylierungsmittel der Formel VI zu einem Diaza-bicyclo[4.1.0]heptenon I bzw. einer cis-2-(p-Acylaminobenzoyl)-cyclopropancarbonsäure der Formel III wird unter an sich üblichen Bedingungen durchgeführt, in der Regel unter Verwendung von wenigstens einer äquimolaren Menge des Acylierungsmittels, zweckmäßig in Gegenwart eines Lösungsmittels und gegebenenfalls in Gegenwart einer Hilfsbase bei Temperaturen zwischen 0 und 160°C, gegebenenfalls bei den Siedetemperaturen der Reaktionsgemische und gegebenenfalls unter Anwendung von Druck. Als Lösungsmittel kommen unter den Reaktionsbedingungen inerte Lösungsmittel, wie aromatische Kohlenwasserstoffe, beispielsweise Toluol oder Xylol, cyclische aliphatische Ether, wie Tetrahydrofuran oder Dioxan, oder Dialkylformamide, wie Dimethylformamid, in Betracht. Hilfsbasen als säurebindende Mittel sind zweckmäßigerweise anorganische Basen, wie Natrium- oder Kaliumcarbonat, Natrium- oder Kaliumhydrogencarbonat, oder tertiäre organische Amine, wie Triethylamin.

Für die Cyclisierung einer cis-2-(p-Acylaminobenzoyl)-cyclopropancarbonsäure der Formel III oder der Aminosäure der Formel IV mit Hydrazin, das bevorzugt als Hydrat eingesetzt wird, zu einem Diaza-bicyclo[4.1.0]heptenon der Formel I oder der Aminoverbindung V wird vorteilhaft unter folgenden Bedingungen gearbeitet: In einem unter den Reaktionsbedingungen inerten Lösungsmittel, insbesondere einem niederen Alkohol, wie Methanol, Ethanol oder Propanol, einem cyclischen aliphatischen Ether, wie Tetrahydrofuran oder Dioxan, oder einem Dialkylformamid, wie Dimethylformamid, und bei Temperaturen von 60 bis 150°C, vorzugsweise 80 bis 120°C. In der Regel werden hierbei je Mol einer Verbindung der Formel III bzw. je Mol der Verbindung der Formel IV 1 bis 1,2 Mol Hydrazin verwendet.

Nach den genannten Verfahren werden beispielsweise die folgenden erfindungsgemäßen Verbindungen erhalten:

2-(p-Aminophenyl)-3,4-diaza-bicyclo[4.1.0]hepten-(2)-on-(5);
2-(p-Formylaminophenyl)-3,4-diaza-bicyclo[4.1.0]hepten-(2)-on-(5);
2-(p-Acetylaminophenyl)-3,4-diaza-bicyclo[4.1.0]hepten-(2)-on-(5);
2-(p-Propionylaminophenyl)-3,4-diaza-bicyclo[4.1.0]hepten-(2)-on-(5);
2-(p-Butyrylaminophenyl)-3,4-diaza-bicyclo[4.1.0]hepten-(2)-on-(5);
2-(p-Isobutyrylaminophenyl)-3,4-diaza-bicyclo[4.1.0]hepten-(2)-on-(5);
2-(p-Valerylaminophenyl)-3,4-diaza-bicyclo[4.1.0]hepten-(2)-on-(5);
2-(p-Isovalerylaminophenyl)-3,4-diaza-bicyclo[4.1.0]hepten-(2)-on-(5);
2-[p-(2-Methylbutyrylamino)-phenyl]-3,4-diaza-bicyclo[4.1.0]hepten-(2)-on-(5);
2-(p-Pivaloylaminophenyl)-3,4-diaza-bicyclo[4.1.0]hepten-(2)-on-(5);
2-(p-Chloracetylaminophenyl)-3,4-diaza-bicyclo[4.1.0]hepten-(2)-on-(5);
2-(p-Bromacetylaminophenyl)-3,4-diaza-bicyclo[4.1.0]hepten-(2)-on-(5);
2-(p-Fluoracetylaminophenyl)-3,4-diaza-bicyclo[4.1.0]hepten-(2)-on-(5);
2-(p-Jodacetylaminophenyl)-3,4-diaza-bicyclo[4.1.0]hepten-(2)-on-(5);
2-[p-(2-Chlorpropionylamino)-phenyl]-3,4-diaza-bicyclo[4.1.0]hepten-(2)-on-(5);
2-[p-(2-Brompropionylamino)-phenyl]-3,4-diaza-bicyclo[4.1.0]hepten-(2)-on-(5);
2-[p-(3-Chlorpropionylamino)-phenyl]-3,4-diaza-bicyclo[4.1.0]hepten-(2)-on-(5);
2-[p-(3-Brompropionylamino)-phenyl]-3,4-diaza-bicyclo[4.1.0]hepten-(2)-on-(5);
2-[p-(2-Chlorbutyrylamino)-phenyl]-3,4-diaza-bicyclo[4.1.0]hepten-(2)-on-(5);
2-[p-(2-Brombutyrylamino)-phenyl]-3,4-diaza-bicyclo[4.1.0]hepten-(2)-on-(5);
2-[p-(4-Chlorbutyrylamino)-phenyl]-3,4-diaza-bicyclo[4.1.0]hepten-(2)-on-(5);
2-[p-(2-Chlorisobutyrylamino)-phenyl]-3,4-diaza-bicyclo[4.1.0]hepten-(2)-on-(5);
2-[p-(2-Bromvalerylamino)-phenyl]-3,4-diaza-bicyclo[4.1.0]-hepten-(2)-on-(5);
2-(p-Chlorpivaloylaminophenyl)-3,4-diaza-bicyclo[4.1.0]hepten-(2)-on-(5);
2-(p-Brompivaloylaminophenyl)-3,4-diaza-bicyclo[4.1.0]hepten-(2)-on-(5);

2-(p-Dichloracetylaminophenyl)-3,4-diaza-bicyclo[4.1.0]hepten-(2)-on-(5);
2-(p-Difluoracetylaminophenyl)-3,4-diaza-bicyclo[4.1.0]hepten-(2)-on-(5);
2-[p-(2,2-Dichlorpropionylamino)-phenyl]-3,4-diaza-bicyclo[4.1.0]hepten-(2)-on-(5);
2-[p-(2,3-Dichlorpropionylamino)-phenyl]-3,4-diaza-bicyclo[4.1.0]hepten-(2)-on-(5);
2-[p-(2,2-Dichlorbutyrylamino)-phenyl]-3,4-diaza-bicyclo[4.1.0]hepten-(2)-on-(5);
2-[p-(2,4-Dichlorbutyrylamino)-phenyl]-3,4-diaza-bicyclo[4.1.0]hepten-(2)-on-(5);

2-(p-Trichloracetylaminophenyl)-3,4-diaza-bicyclo[4.1.0]hepten-(2)-on-(5);
2-(p-Trifluoracetylaminophenyl)-3,4-diaza-bicyclo[4.1.0]hepten-(2)-on-(5);
2-(p-Chlordifluoracetylaminophenyl)-3,4-diaza-bicyclo[4.1.0]hepten-(2)-on-(5);

2-(p-Cyclopropylcarbonylaminophenyl)-3,4-diaza-bicyclo[4.1.0]hepten-(2)-on-(5);
2-[p-(1-Methylcyclopropylcarbonylamino)-phenyl]-3,4-diaza-bicyclo[4.1.0]hepten-(2)-on-(5);
2-[p-(2-Methylcyclopropylcarbonylamino)-phenyl]-3,4-diaza-bicyclo[4.1.0]hepten-(2)-on-(5);
2-[p-(2,2-Dimethylcyclopropylcarbonylamino)-phenyl]-3,4-diaza-bicyclo[4.1.0]-hepten-(2)-on-(5);

2-[p-(1-Chlorcyclopropylcarbonylamino)-phenyl]-3,4-diaza-bicyclo[4.1.0]hepten-(2)-on-(5);
2-[p-(2-Bromcyclopropylcarbonylamino)-phenyl]-3,4-diaza-bicyclo[4.1.0]hepten-(2)-on-(5);
2-[p-(2,2-Dichlorcyclopropylcarbonylamino)-phenyl]-3,4-diaza-bicyclo[4.1.0]-hepten-(2)-on-(5);
2-[p-(2,2-Dichlor-1-methylcyclopropylcarbonylamino)-phenyl]-3,4-diaza-bicyclo[4.1.0]-hepten-(2)-on-(5);
2-(p-Cyclobutylcarbonylaminophenyl)-3,4-diaza-bicyclo[4.1.0]hepten-(2)-on-(5);
2-[p-(2-Methylcyclobutylcarbonylamino)-phenyl]-3,4-diaza-bicyclo[4.1.0]hepten-(2)-on-(5);
2-[p-(1-Chlorcyclobutylcarbonylamino)-phenyl]-3,4-diaza-bicyclo[4.1.0]hepten-(2)-on-(5);
2-[p-(2-Chlorcyclobutylcarbonylamino)-phenyl]-3,4-diaza-bicyclo[4.1.0]hepten-(2)-on-(5);
2-[p-(3-Chlorcyclobutylcarbonylamino)-phenyl]-3,4-diaza-bicyclo[4.1.0]hepten-(2)-on-(5);
2-(p-Cyclopentylcarbonylaminophenyl)-3,4-diaza-bicyclo[4.1.0]hepten-(2)-on-(5);

2-(p-Acryloylaminophenyl)-3,4-diaza-bicyclo[4.1.0]hepten-(2)-on-(5);
2-(p-Crotonoylaminophenyl)-3,4-diaza-bicyclo[4.1.0]hepten-(2)-on-(5);
2-(p-Methacryloylaminophenyl)-3,4-diaza-bicyclo[4.1.0]hepten-(2)-on-(5);
2-[p-(But-3-enoylamino)-phenyl]-3,4-diaza-bicyclo[4.1.0]hepten-(2)-on-(5).

Es sei darauf hingewiesen, daß die erfindungsgemäßen Verbindungen der Formel I und auch die Verbindung der Formel V in den Stellungen 1 und 6 des 3,4-Diaza-bicyclo[4.1.0]hepten-(2)-on-(5)-Ringes asymmetrische Kohlenstoffatome aufweisen und als Racemate erhalten werden. Die vorliegende Erfindung soll die Enantiomeren mit einschließen. Sie können nach an sich üblichen Verfahren mit einer optisch aktiven Säure, wie Dibenzoylweinsäure oder Campher-10-sulfonsäure, getrennt werden. Gegebenenfalls erfolgt die Trennung bei der Herstellung bereits an einer Zwischenstufe.

Die erfindungsgemäßen 2-Aryl-3,4-diaza-bicyclo[4.1.0]-heptenone der Formel I zeichnen sich durch eine starke thrombozytenaggregationshemmende Wirkung und durch eine starke blutdrucksenkende Wirkung aus. Sie sind demnach als Antihypertensiva und zur Prophylaxe und Therapie thrombo-embolischer Erkrankungen geeignet.

Gegenstand der vorliegenden Erfindung sind demnach auch therapeutische Mittel oder Zubereitungen, die neben pharmazeutisch üblichen Träger- und Verdünnungsmitteln eine Verbindung der Formel I als Wirkstoff enthalten, sowie die Verwendung dieser Verbindungen zu therapeutischen Zwecken bei der Behandlung von hohem Blutdruck oder thrombo-embolischer Erkrankungen.

Zur Untersuchung der pharmakodynamischen Eigenschaften der erfindungsgemäßen Produkte wurden folgende Methoden verwendet:

### 1. Hemmung der durch Collagen induzierten Aggregation von Thrombozyten des Menschen in vitro

Thrombozytenreiches Plasma wird durch Zentrifugation (300 g, 10 min Dauer bei 4°C) von venösem Citratblut gewonnen. Die photometrische Messung der Thrombozytenaggregation erfolgt unter Zusatz von $MgCl_2$ (Endkonzentration 10 mmol/l) und von Collagen Stago (Endkonzentration 0,02 mg/ml) im Born-Aggregometer Mk 3. Als Aggregationsmaß findet die maximale Extinktionsänderung/sec Verwendung.

Die Prüfung der aggregationshemmenden Wirksamkeit der Substanzen wird nach einer Inkubationszeit von 10 min vorgenommen.

Als EC 50% wird die Konzentration bestimmt, welche eine 50%ige Hemmung der Aggregation verursacht.

### 2. Hemmung der durch Collagen induzierten Aggregation von Thrombozyten der Ratte ex vivo

Die Substanzen werden Gruppen von 10–15 männlichen Sprague-Dawley-Ratten (200–250 g) oral appliziert. 1 Stunde nach der Applikation wird in Ether-Narkose Blut entnommen und durch Zentrifugation thrombozytenreiches Plasma gewonnen. Die Messung der Aggregation nach Collagen erfolgt wie oben angegeben. Als ED 33% wird die Dosis bestimmt, welche die durch Collagen induzierte Thrombozytenaggregation um 33% hemmt.

### 3. Blutdrucksenkende Wirkung an der narkotisierten Ratte

Zur Testung der blutdrucksenkenden Wirkung erhalten Gruppen von 3–5 männlichen Sprague-Dawley-Ratten (240–280 g) in Urethan-Narkose (1,78 mg/kg i.p.) die Substanzen intraperitoneal appliziert.

Die Messung des Blutdrucks in der Arteria carotis erfolgt über Statham-Transducer. Als ED 20% wird die Dosis bestimmt, welche den mittleren Carotisblutdruck um 20% senkt.

### 4. Antihypertensive Wirkung an der spontan hypertonen Ratte

Die Substanzen werden Gruppen von 4—8 männlichen spontan hypertonen Okamoto-Ratgten (270—340 g) oral appliziert. Der systolische Blutdruck wird vor und 2 Stunden nach der Applikation unblutig mit Hilfe von Piezokristallaufnehmern gemessen.

Als ED 20% wird unter Berücksichtigung der Werte unbehandelter Kontrolltiere die Dosis bestimmt, welche den systolischen Druck um 20% senkt.

Die Berechnung der wirksamen Dosen bzw. Konzentrationen erfolgte aus den linearen Beziehungen zwischen den Logarithmen der Dosen bzw. Konzentrationen und der Wirkung mit Hilfe der Regressionsanalyse.

Als Referenzsubstanz für die Hemmung der Thrombozytenaggregation diente Acetylsalicylsäure, für die blutdrucksenkende Wirkung Dihydralazin.

Die Ergebnisse der Tabelle 1 zeigen für die erfindungsgemäßen Verbindungen eine außerordentlich starke Hemmung der durch Collagen induzierten Aggregation von Thrombozyten des Menschen. Die Wirkung ist 73 bis 3530 mal stärker als die des bekannten aggregationshemmenden Pharmakons Acetylsalicylsäure.

Außer der Hemmung der Thrombozytenaggregation tritt eine blutdrucksenkende Wirkung von unterschiedlicher Stärke auf. An der Ratte wirken einzelne Verbindungen wesentlich stärker als das bekannte Antihypertonicum Dihydralazin. So erreichen die Beispiele Nr. 7 und Nr. 5 die 7,25- bzw. 2,52fache Wirksamkeit von Dihydralazin, wie aus der Tabelle 2 ersichtlich ist.

Von besonderer Bedeutung für die pharmakotherapeutische Anwendung ist die Tatsache, daß die Thrombozytenaggregation auch nach oraler Applikation in vivo stark ausgeprägt ist und den Effekt von Acetylsalicylsäure bei weitem übertrifft, wie aus der Tabelle 3 (Beispiel 1, 5, 7, 14) hervorgeht.

In gleicher Weise läßt sich an spontan hypertonen Ratten nach oraler Applikation der Beispiele 1, 5, 7 und 14 eine antihypertensive Wirkung nachweisen (Tabelle 3).

Tabelle 1 — Hemmung der durch Collagen induzierten Thrombozyten-aggregation in vitro

| Verbindung Bsp. Nr. | Aggregationshemmung[1]) EC 50% | R.W.[2]) |
|---|---|---|
| 1 | 0,14 | 3530 |
| 4 | 6,81 | 73 |
| 5 | 0,28 | 1760 |
| 6 | 0,95 | 518 |
| 7 | 5,57 | 89 |
| 8 | 0,38 | 1310 |
| 9 | 1,87 | 264 |
| 10 | 4,88 | 101 |
| 12 | 3,99 | 124 |
| 13 | 3,52 | 140 |
| 14 | 0,99 | 499 |
| 15 | 2,27 | 218 |
| 16 | 3,47 | 142 |
| 17 | 2,99 | 165 |
| 18 | 3,20 | 154 |
| 19 | 1,80 | 274 |
| 20 | 1,91 | 259 |
| 21 | 1,42 | 348 |
| 22 | 2,12 | 233 |
| Acetylsalicylsäure | 494 | 1,00 |

[1]) Menschliche Thrombozyten in vitro. EC 50% (mg/l) = Konzentration, welche die durch Collagen induzierte Aggregation um 50% hemmt.
[2]) R.W. = Relative Wirksamkeit; Acetylsalicylsäure = 1,00.

Tabelle 2 – Blutdrucksenkende Wirkung

| Verbindung Bsp. Nr. | Blutdrucksenkung[1]) ED 20% | R.W.[2]) |
|---|---|---|
| 1 | 0,193 | 1,75 |
| 4 | 0,253 | 1,33 |
| 5 | 0,134 | 2,52 |
| 6 | 0,299 | 1,13 |
| 7 | 0,0465 | 7,25 |
| 8 | 0,298 | 1,13 |
| 9 | 1,88 | 0,18 |
| 10 | 0,402 | 0,84 |
| 12 | 0,900 | 0,37 |
| 13 | 0,688 | 0,49 |
| 14 | 0,278 | 1,21 |
| 15 | 6,81 | 0,05 |
| 16 | 2,96 | 0,11 |
| 17 | 1,50 | 0,23 |
| 18 | 10,0 | 0,03 |
| 19 | 0,649 | 0,52 |
| 20 | 0,786 | 0,43 |
| 21 | 0,398 | 0,85 |
| 22 | 1,71 | 0,20 |
| Dihydralazin | 0,337 | 1,0 |

[1]) Ratte, Urethan-Narkose, Appl. i.p. ED 20% (mg/kg) = Dosis, welche den Blutdruck um 20% senkt.
[2]) R.W. = Relative Wirksamkeit; Dihydralazin = 1,00.

Tabelle 3 – Hemmung der Thrombozytenaggregation und antihypertensive Wirkung nach oraler Applikation

| Verbindung Bsp. Nr. | Hemmung der Thrombozytenaggregation[1] | | Antihypertensive Wirkung[2] | |
|---|---|---|---|---|
| | ED 33% | R.W. | ED 20% | R.W. |
| 1 | 30,7 | 4,36 | 8,65 | 0,79 |
| 7 | 2,50 | 53,60 | 2,78 | 2,46 |
| 5 | 1,48 | 90,54 | 10,0 | 0,69 |
| 14 | 3,74 | 35,83 | 10,0 | 0,69 |
| Acetylsalicylsäure | 134 | 1,00 | – | – |
| Dihydralazin | – | – | 6,85 | 1,00 |

[1]) Ratte, Appl. per os, ED 33% (mg/kg) = Dosis, welche die durch Collagen induzierte Aggregation um 33% hemmt.
R.W. = Relative Wirksamkeit, Acetylsalicylsäure = 1,00.

[2]) Spontan hypertone Ratte, Appl. per os, ED 20% (mg/kg) = Dosis, welche den Blutdruck im Vergleich zur Kontrollgruppe um 20% senkt. R.W. Dihydralazin = 1,00.

Bezüglich ihrer pharmakologischen Wirksamkeit können von den Verbindungen der Formel I beispielsweise hervorgehoben werden:

2-(p-Formylaminophenyl)-3,4-diaza-bicyclo[4.1.0]hepten-(2)-on-(5);
2-(p-Butyrylaminophenyl)-3,4-diaza-bicyclo[4.1.0]hepten-(2)-on-(5);
2-[p-(2-Methylbutyrylamino)-phenyl]-3,4-diaza-bicyclo[4.1.0]hepten-(2)-on-(5);
2-[p-(3-Chlorpropionylamino)-phenyl]-3,4-diaza-bicyclo[4.1.0]hepten-(2)-on-(5);
2-[p-(4-Chlorbutyrylamino)-phenyl]-3,4-diaza-bicyclo[4.1.0]hepten-(2)-on-(5);
2-(p-Dichloracetylaminophenyl)-3,4-diaza-bicyclo[4.1.0]hepten-(2)-on-(5);
2-[p-(2-Methylcyclopropylcarbonylamino)-phenyl]-3,4-diaza-bicyclo[4.1.0]hepten-(2)-on-(5);
2-[p-(2,2-Dichlor-1-methylcyclopropylcarbonylamino)-phenyl]-3,4-diaza-bicyclo[4.1.0]-hepten-(2)-on-(5);
2-(p-Isobutyrylaminophenyl)-3,4-diaza-bicyclo[4.1.0]hepten-(2)-on-(5);
2-(p-Brompivaloylaminophenyl)-3,4-diaza-bicyclo[4.1.0]hepten-(2)-on-(5);
2-[p-(2,2-Dichlorpropionylamino)-phenyl]-3,4-diaza-bicyclo[4.1.0]hepten-(2)-on-(5);

Als Einzelverbindungen können besonders hervorgehoben werden:

2-(p-Acetylaminophenyl)-3,4-diaza-bicyclo[4.1.0]hepten-(2)-on-(5);
2-(p-Propionylaminophenyl)-3,4-diaza-bicyclo[4.1.0]hepten-(2)-on-(5);
2-(p-Chloracetylaminophenyl)-3,4-diaza-bicyclo[4.1.0]hepten-(2)-on-(5);
2-[p-(2-Chlorpropionylamino)-phenyl]-3,4-diaza-bicyclo[4.1.0]hepten-(2)-on-(5);
2-(p-Cyclopropylcarbonylaminophenyl)-3,4-diaza-bicyclo[4.1.0]hepten-(2)-on-(5);
2-(p-Acryloylaminophenyl)-3,4-diaza-bicyclo[4.1.0]hepten-(2)-on-(5);
2-(p-Cyclobutylcarbonylaminophenyl)-3,4-diaza-bicyclo[4.1.0]hepten-(2)-on-(5).

Die therapeutischen Mittel oder Zubereitungen werden mit den pharmazeutisch üblichen Trägerstoffen oder Verdünnungsmitteln und den üblicherweise verwendeten pharmazeutisch-technischen Hilfsstoffen entsprechend der gewünschten Applikationsart mit einer geeigneten Dosierung in bekannter Weise hergestellt. Dabei kommen beim Menschen Dosen von 1 bis 100 mg, bevorzugt 5 bis 50 mg, in Betracht, wobei die orale Applikation bevorzugt ist.

Darreichungsformen, die zur oralen Applikation geeignet sind, sind beispielsweise Tabletten, Filmtabletten, Dragees, Kapseln, Pillen, Pulver, Lösungen, Suspensionen oder Depotformen.

Zur praktischen Anwendung werden die erfindungsgemäß zu verwendenden Verbindungen mit den in der galenischen Pharmazie üblichen festen oder flüssigen Trägerstoffen verarbeitet. Die entsprechenden Tabletten können beispielsweise durch Mischen des Wirkstoffs mit bekannten Hilfsstoffen, beispielsweise inerten Verdünnungsmitteln, wie Dextrose, Zucker, Sorbit, Polyvinylpyrrolidon, Mannit, Calciumcarbonat, Calciumphosphat oder Milchzucker, Sprengmitteln, wie Mais, Stärke, Alginsäure oder Polyvinylpyrrolidon, Bindemitteln wie Stärke oder Gelatine, Gleitmitteln, wie Magnesiumstearat

oder Talkum, und/oder Mitteln zur Erzielung des Depoteffektes, wie Carboxypolymethylen, Carboxymethylcellulose, Celluloseacetatphthalat oder Polyvinylacetat, erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Kollidon oder Schellack, Gummi arabicum, Talk, Titandioxid oder Zucker, hergestellt werden. Dabei kann auch die Drageehülle aus mehreren Schichten bestehen, wobei die oben bei den Tabletten erwähnten Hilfsstoffe verwendet werden können.

Die Herstellung der neuen 2-Aryl-3,4-diaza-bicyclo[4.1.0]hepten-(2)-one-(5) wird durch die folgenden Beispiele näher erläutert.

Beispiel 1

a) Zu 29,7 g (0,22 mol) wasserfreiem Aluminiumchlorid in 100 ml Schwefelkohlenstoff gibt man unter Rühren bei Raumtemperatur portionsweise zuerst 11,1 g (82,1 mmol) Acetanilid und anschließend 10,0 g (89,2 mmol) cis-Cyclopropan-1,2-dicarbonsäureanhydrid. Das Gemisch wird 4 Stunden am Rückfluß gehalten und dann 2 Tage bei Raumtemperatur stehen gelassen. Nach dem Abdekantieren des Schwefelkohlenstoffs wird das Reaktionsgemisch in 200 g Eis und 50 ml konzentrierte Salzsäure eingetragen. Die ausgefallene Festsubstanz wird abgesaugt, mit Wasser gewaschen und aus Methanol umkristallisiert. Man erhält 5,1 g (25 % d. Th.) cis-2-(p-Acetylaminobenzoyl)-cyclopropancarbonsäure, fast farblose Kristalle, Schmelzpunkt 227 bis 228°C.

Analyse für $C_{13}H_{13}NO_4$:

ber.: C 63,2 H 5,3 N 5,7 O 25,9 %
gef.: C 63,0 H 5,5 N 5,9 O 24,9 %

b) Zu 120 g (0,90 mol) wasserfreiem Aluminiumchlorid gibt man unter Rühren innerhalb weniger Minuten tropfenweise 18 ml (0,23 mol) Dimethylformamid, wobei eine stark exotherme Reaktion eintritt. Man fügt dann bei 60 bis 70°C portionsweise ein Gemisch aus 12,0 g (88,8 mmol) Acetanilid und 10,0 g (89,2 mmol) cis-Cyclopropan-1,2-dicarbonsäureanhydrid hinzu und rührt anschließend noch 1 Stunde bei 70°C nach. Aufarbeitung wie im Beispiel 1 a ergibt nach Umkristallisation aus Methanol 13,7 g (62 % d.Th.) cis-2-(p-Acetylaminobenzoyl)-cyclopropancarbonsäure, beige Kristalle (identisch mit der Verbindung aus Beispiel 1 a).

c) 6,0 g (24,3 mmol) cis-2-(p-Acetylaminobenzoyl)-cyclopropancarbonsäure werden mit 1,33 g (26,6 mmol) Hydrazinhydrat und 150 ml Ethanol 6 Stunden am Rückfluß gehalten. Nach dem Absaugen bei 0°C und dem Trocknen im Vakuum bei 50°C isoliert man 5,0 g (85 % d.Th.) 2-(p-Acetylaminophenyl)-3,4-diaza-bicyclo[4.1.0]hepten-(2)-on-(5), farblose Kristalle, Schmelzpunkt 269 bis 270°C (umkristallisiert aus Methanol/Wasser).

Analyse für $C_{13}H_{13}N_3O_2$:

ber.: C 64,2 H 5,4 N 17,3 O 13,2 %;
gef.: C 64,0 H 5,5 N 17,3 O 13,5 %.

Beispiel 2

a) 25 g (103 mmol) 2-(p-Acetylaminophenyl)-3,4-diaza-bicyclo[4.1.0]hepten-(2)-on-(5) (s. Beispiel 1 c) werden mit 215 ml Methanol und 215 ml 10 n Natronlauge 3 Stunden am Rückfluß gehalten. Anschließend zieht man das Methanol im Vakuum ab. Der Rückstand wird mit 500 ml Wasser versetzt und mit verdünnter Salzsäure auf pH 4 eingestellt. Nach dem Absaugen und dem Trocknen im Vakuum bei 50°C isoliert man 19,3 g (93 % d.Th.) 2-(p-Aminophenyl)-3,4-diaza-bicyclo[4.1.0]hepten-(2)-on-(5), fsst farblose Kristalle, Schmelzpunkt 230 bis 231 °C (umkristallisiert aus Methanol).

Analyse für $C_{11}H_{11}N_3O$:

ber.: C 65,7 H 5,5 N 20,9 %,
gef.: C 65,3 H 5,5 N 21,1 %;

b) 6,0 g (29,8 mmol) der oben erhaltenen Aminoverbindung werden bei 50°C in 50 ml 10%iger wäßriger Salzsäure gelöst. Man kühlt auf 0°C ab und saugt die ausgefallene Festsubstanz ab. Nach dem Umkristallisieren aus Methanol/Ether isoliert man 5,2 g (73% d. Th.) 2-(p-Aminophenyl)-3,4-diaza-bicyclo[4.1.0]hepten-(2)-on-(5)-hydrochlorid, fast farblose Kristalle, Schmelzpunkt 288 bis 290°C.

Analyse für $C_{11}H_{12}ClN_3O$:

ber.: C 55,6 H 5,1 Cl 14,9 N 17,7 O 6,7%;
gef.: C 55,6 H 5,2 Cl 14,8 N 17,6 O 6,9%.

Beispiel 3

a) 95 g (384 mmol) cis-2-(p-Acetylaminobenzoyl)-cyclopropansäure (s. Beispiel 1 b) werden mit 600 ml 6 n Salzsäure circa 30 Minuten bei 90 bis 95°C gehalten. Die Lösung wird anschließend bei 10°C mit verdünnter Natronlauge auf pH 4 eingestellt. Die ausgefallene Festsubstanz wird abgesaugt und mit Wasser gewaschen. Nach der Umkristallisation aus Wasser isoliert man 46,4 g (59% d. Th.) cis-2-(p-Aminobenzoyl)-cyclopropancarbonsäure, hellgraue Kristalle, Schmelzpunkt 190 bis 191°C.

Analyse für $C_{11}H_{11}NO_3$:

ber.: C 64,4 H 5,4 N 6,8%,
gef.: C 64,4 H 5,3 N 7,0%;

b) 20 g (97,5 mmol) cis-2-(p-Aminobenzoyl)-cyclopropancarbonsäure werden mit 5,9 g (118 mmol) Hydrazinhydrat und 100 ml Ethanol 5 Stunden am Rückfluß gehalten. Nach dem Absaugen bei 10°C und dem Trocknen im Vakuum bei 50°C erhält man 18,6 g (95% d. Th.) 2-(p-Aminophenyl)-3,4-diaza-bicyclo[4.1.0]hepten-(2)-on-(5), hellgelbe Kristalle (identisch mit der Verbindung aus Beispiel 2 a).

Beispiel 4

6,0 g (29,8 mmol) 2-(p-Aminophenyl)-3,4-diaza-bicyclo[4.1.0]hepten-(2)-on-(5) (s. Beispiel 2 a) werden mit 30 ml Ameisensäure 1 Stunde am Rückfluß gehalten. Man gießt die Lösung auf 500 ml Wasser, saugt den ausgefallenen Niederschlag ab, wäscht mit Wasser und kristallisiert aus Methanol/Wasser um. Man isoliert 4,6 g (67% d. Th.) 2-(p-Formylaminophenyl)-3,4-diaza-bicyclo[4.1.0]hepten-(2)-on-(5), fast farblose Kristalle, Schmelzpunkt 239 bis 240°C.

Analyse für $C_{12}H_{11}N_3O_2$:

ber.: C 62,9 H 4,8 N 18,3 O 14,0%;
gef.: C 62,7 H 4,9 N 18,6 O 14,3%.

Beispiel 5

6,0 g (29,9 mmol) 2-(p-Aminophenyl)-3,4-diaza-bicyclo[4.1.0]hepten-(2)-on-(5) (s. Beispiel 3 b) und 4,2 g (45,4 mmol) Propionylchlorid werden mit 100 ml wasserfreiem Toluol 6 Stunden bei 80°C gehalten. Man saugt bei 10°C ab, wäscht zuerst mit Toluol, dann mit Wasser und trocknet im Vakuum bei 50°C. Man erhält 6,5 g (83% d. Th.) 2-(p-Propionylaminophenyl)-3,4-diaza-bicyclo[4.1.0]hepten-(2)-on-(5)-viertelhydrat, fast farblose Kristalle, Schmelzpunkt 252 bis 253°C (umkristallisiert aus Dimethylformamid/Wasser).

Analyse für $C_{14}H_{15}N_3O_2 \cdot 1/4\,H_2O$:

ber.: C 64,2 H 6,0 N 16,1%;
gef.: C 64,2 H 5,8 N 16,4%.

## Beispiel 6

Wird im Beispiel 5 anstatt Propionylchlorid Chloracetylchlorid (3,7 g [32,8 mmol]) eingesetzt, so erhält man nach Umkristallisation aus Wasser 7,5 g (91 % d. Th.) 2-(p-Chloracetylaminophenyl)-3,4-diaza-bicyclo[4.1.0]hepten-(2)-on-(5), hellgelbe Kristalle, Schmelzpunkt 220 bis 221 °C.

Analyse für $C_{13}H_{12}ClN_3O_2$:

ber.: C 56,2 H 4,4 Cl 12,8 N 15,1 O 11,5 %;
gef.: C 55,8 H 4,2 Cl 12,7 N 15,2 O 12,2 %.

## Beispiel 7

Führt man das Beispiel 5 unter Verwendung von Cyclopropancarbonsäurechlorid (4,65 g [44,5 mmol]) anstelle von Propionylchlorid durch, so isoliert man nach der Umkristallisation aus Dimethylformamid/Wasser 4,5 g (54 % d. Th.) 2-(p-Cyclopropylcarbonylaminophenyl)-3,4-diaza-bicyclo[4.1.0]hepten-(2)-on-(5)-hemihydrat, beige Kristalle, Schmelzpunkt 277 bis 279 °C.

Analyse für $C_{15}H_{15}N_3O_2 \cdot 1/2\,H_2O$:

ber.: C 64,7 H 5,8 N 15,1 %;
gef.: C 65,0 H 5,4 N 15,3 %.

## Beispiel 8

Analog Beispiel 5 werden 6,0 g (29,8 mmol) 2-(p-Aminophenyl)-3,4-diaza-bicyclo[4.1.0]hepten-(2)-on-(5) (s. Beispiel 2a) mit 4,05 g (44,7 mmol) Acrylsäurechlorid in Toluol umgesetzt. Man saugt bei 10 °C ab, wäscht zuerst mit Toluol, dann mit Wasser und kristallisiert aus Dimethylformamid/Wasser um. Man isoliert 3,5 g (45 % d. Th.) 2-(p-Acryloylaminophenyl)-3,4-diaza-bicyclo[4.1.0]hepten-(2)-on-(5)-viertelhydrat, fast farblose Kristalle, Schmelzpunkt 240 bis 241 °C.

Analyse für $C_{14}H_{13}N_3O_2 \cdot 1/4\,H_2O$:

ber.: C 64,7 H 5,2 N 16,2 O 13,9 %;
gef.: C 64,5 H 4,9 N 16,2 O 13,0 %.

In der folgenden Tabelle sind die Ausführungsbeispiele 9 bis 22 zusammengestellt. Die Herstellung dieser Diaza-bicyclo[4.1.0]heptenone erfolgte nach der im Beispiel 5 beschriebenen Methode.

Tabelle

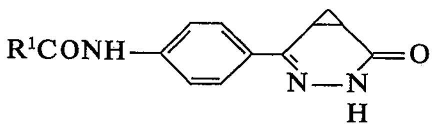

(I)

| Bsp. | $R^1$ | Schmp. [°C] | Analyse (%) | C | H | Cl | N |
|---|---|---|---|---|---|---|---|
| 9 | $CH_3-CH_2-CH_2-$ | 241–242 | ber.: | 66,4 | 6,3 | – | 15,5 |
| | | (DMF/Wasser) | gef.: | 65,9 | 6,4 | – | 16,0 |
| 10 | 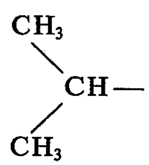 | 277–278 (· 1/2 $H_2O$) | ber.: | 64,3 | 6,5 | – | 15,0 |
| | | (DMF/Wasser) | gef.: | 64,6 | 6,3 | – | 15,2 |
| 11 | $CH_3-CH_2-CH_2-CH_2-$ | 207–208 | ber.: | 67,3 | 6,7 | – | 14,7 |
| | | (DMF/Wasser) | gef.: | 66,9 | 6,8 | – | 14,7 |
| 12 | 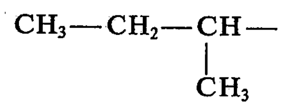 | 227–228 | ber.: | 67,3 | 6,7 | – | 14,7 |
| | | (DMF/Wasser) | gef.: | 67,1 | 6,7 | – | 15,0 |
| 13 | $Cl-CH_2-CH_2-$ | Ab 215 mit Zers. | ber.: | 57,6 | 4,8 | 12,2 | 14,4 |
| | | (DMF/Wasser) | gef.: | 57,3 | 5,0 | 11,5 | 14,5 |

| Bsp. | R[1] | Schmp. [°C] | Analyse (%) | C | H | Cl | N | O |
|---|---|---|---|---|---|---|---|---|
| 14 | 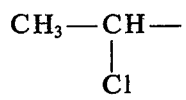 | 236–237 | ber.: | 57,6 | 4,8 | 12,2 | 14,4 | 11,0 |
| | | (Ethanol) | gef.: | 57,6 | 5,2 | 12,2 | 14,3 | 11,4 |
| 15 | $Cl—CH_2—CH_2—CH_2—$ | 206–207 | ber.: | 58,9 | 5,3 | 11,6 | 13,7 | – |
| | | (DMF/Wasser) | gef.: | 59,1 | 5,3 | 11,2 | 14,0 | – |
| 16 | 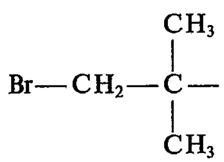 | 211–212 | ber.: | 52,8 | 5,0 | 21,9 | 11,5 | – |
| | | ($CH_3OH$/Aceton) | gef.: | 53,1 | 5,0 | 20,9 | 12,0 | – |
| 17 | 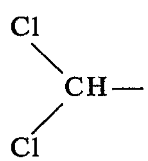 | Ab 251 mit Zers. | ber.: | 50,0 | 3,6 | 22,7 | 13,5 | – |
| | | (Propanol) | gef.: | 50,1 | 3,9 | 22,4 | 13,7 | – |
| 18 | 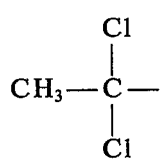 | 235–237 (Zers.) | ber.: | 51,6 | 4,0 | 21,7 | 12,9 | – |
| | | (Propanol) | gef.: | 51,8 | 4,1 | 21,2 | 13,3 | – |
| 19 | 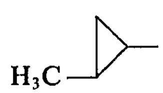 | 268–269 | ber.: | 67,8 | 6,0 | – | 14,8 | – |
| | | (Ethanol) | gef.: | 67,4 | 6,0 | – | 14,8 | – |

Fortsetzung

| Bsp. | $R^1$ | Schmp. [°C] | Analyse (%) | C | H | Cl | N | O |
|---|---|---|---|---|---|---|---|---|
| 20 | 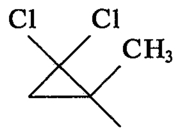 | 238–239 (Zers.) | ber.: | 54,6 | 4,3 | 20,1 | 11,9 | – |
| | | (DMF/Wasser) | gef.: | 54,7 | 4,3 | 19,6 | 12,2 | – |
| 21 | 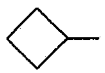 | 276–277 | ber.: | 67,8 | 6,0 | – | 14,8 | – |
| | | (Ethanol/Wasser) | gef.: | 67,4 | 5,9 | – | 14,6 | – |
| 22 | $CH_3—CH=CH—$ | 232–233 ( · 1/4 $H_2O$) | ber.: | 65,8 | 5,7 | – | 15,3 | 13,1 |
| | cis : trans ≃ 10 : 90 (Methanol/Wasser) | | gef.: | 65,7 | 5,8 | – | 15,5 | 12,7 |

### Beispiel 23

a) 5,0 (24,4 mmol) cis-2-(p-Aminobenzoyl)-cyclopropancarbonsäure (s. Beispiel 3 a) werden mit 50 ml Propionsäureanhydrid 30 Minuten bei 80°C gehalten. Anschließend wird die Lösung auf Eiswasser gegossen. Das dabei abgeschiedene Öl wird über Nacht bei Raumtemperatur in der wäßrigen Phase stehen gelassen, wobei es erstarrt. Man saugt ab, wäscht mit Wasser und kristallisiert aus Essigester/Petrolether um. Man isoliert 1,9 g (30 % d. Th.) cis-2-(p-Propionylaminobenzoyl)-cyclopropancarbonsäure, farblose Kristalle, Schmelzpunkt 182 bis 183°C.

Analyse für $C_{14}H_{15}NO_4$:

ber.: C 64,4 H 5,8 N 5,4 %;
gef.: C 64,3 H 6,0 N 5,3 %.

b) 1,0 g (3,8 mmol) cis-2-(p-Propionylaminobenzoyl)-cyclopropancarbonsäure wird mit 0,22 g (4,4 mmol) Hydrazinhydrat und 20 ml Ethanol 5 Stunden am Rückfluß gekocht. Nach dem Absaugen bei 25°C und dem Trocknen im Vakuum bei 50°C erhält man 0,8 g (81 % d. Th.) 2-(p-Propionylaminophenyl)-3,4-diaza-bicyclo[4.1.0]hepten-(2)-on-(5), fast farblose Kristalle, Schmelzpunkt 268 bis 270°C.

Analyse für $C_{14}H_{15}N_3O_2$:

ber.: C 65,4 H 5,9 N 16,3 %;
gef.: C 65,3 H 5,9 N 16,5 %.

### Beispiel 24

3,0 g (14,9 mmol) 2-(p-Aminophenyl)-3,4-diaza-bicyclo[4.1.0]hepten-(2)-on-(5) werden mit 2,0 g (17,5 mmol) Difluoracetylchlorid und 100 ml absolutem Tetrahydrofuran zuerst 3 Stunden bei 0 bis 5°C und dann noch 20 Stunden bei Raumtemperatur gehalten. Man saugt bei 10°C ab, wäscht mit Wasser und kristallisiert aus Methanol um. Man erhält 2,7 g (63 % d. Th.) 2-(p-Difluoracetylaminophenyl)-3,4-diaza-bicyclo[4.1.0]hepten-(2)-on-(5)-hemihydrat, farblose Kristalle, Schmelzpunkt 223 bis 224°C.

Analyse für $C_{13}H_{11}F_2N_3O_2 \cdot 1/2\,H_2O$:

ber.: C 54,2 H 4,2 F 13,2 N 14,6 %;
gef.: C 54,3 H 4,3 F 13,2 N 14,7 %.

### Beispiel 25

5,0 g (24,8 mmol) 2-(p-Aminophenyl)-3,4-diaza-bicyclo[4.1.0]hepten-(2)-on-(5) werden mit 30 ml Trifluoressigsäureanhydrid 1 Stunde bei Rückflußtemperatur gehalten. Man saugt bei 10°C ab, wäscht zuerst mit Toluol, dann mit Wasser und kristallisiert aus Dimethylformamid/Wasser um. Man isoliert 5,1 g (69 % d. Th.) 2-(p-Trifluoracetylaminophenyl)-3,4-diaza-bicyclo[4.1.0]hepten-(2)-on-(5), farblose Kristalle, Schmelzpunkt 242 bis 243°C.

Analyse für $C_{13}H_{10}F_3N_3O_2$:

ber.: C 52,5 H 3,4 F 19,2 N 14,1 %;
gef.: C 52,5 H 3,6 F 18,9 N 14,3 %.

### Beispiel 26

6,0 g (29,8 mmol) 2-(p-Aminophenyl)-3,4-diaza-bicyclo[4.1.0]hepten-(2)-on-(5) werden mit 4,2 g (35,4 mmol) 1-Methylcyclopropancarbonsäurechlorid und 150 ml absolutem Tetrahydrofuran 7 Stunden am Rückfluß gehalten. Man saugt bei 10°C ab, wäscht mit Wasser und kristallisiert zweimal aus Dimethylformamid/Wasser um. Man erhält 4,5 g (53 % d. Th.) 2-[p-(1-Methyl-

cyclopropylcarbonylamino)-phenyl]-3,4-diaza-bicyclo[4.1.0]hepten-(2)-on-(5), hellbeige Kristalle, Schmelzpunkt 253 bis 255°C.

Analyse für $C_{16}H_{17}N_3O_2$:

ber.: C 67,8 H 6,0 N 14,8%;
gef.: C 67,5 H 6,0 N 15,1%.

Beispiel 27

6,0 g (29,8 mmol) 2-(p-Aminophenyl)-3,4-diaza-bicyclo[4.1.0]hepten-(2)-on-(5) werden mit 4,7 g (35,4 mmol) 2,2-Dimethylcyclopropancarbonsäurechlorid und 150 ml absolutem Tetrahydrofuran 10 Stunden bei Rückflußtemperatur gehalten. Die Reaktionslösung wird anschließend eingeengt. Nach dem Umkristallisieren des Rückstands aus Dimethylformamid/Wasser isoliert man 6,7 g (75% d. Th.) 2-[p-(2,2-Dimethylcyclopropylcarbonylamino)-phenyl]-3,4-diaza-bicyclo[4.1.0]hepten-(2)-on-(5), fast farblose Kristalle, Schmelzpunkt 250 bis 252°C.

Analyse für $C_{17}H_{19}N_3O_2$:

ber.: C 68,7 H 6,4 N 14,1%;
gef.: C 68,3 H 6,4 N 13,9%.

Beispiel 28

5,0 g (24,8 mmol) 2-(p-Aminophenyl)-3,4-diaza-bicyclo[4.1.0]hepten-(2)-on-(5) werden mit 4,5 g (32,4 mmol) 1-Chlorcyclopropancarbonsäurechlorid und 100 ml absolutem Tetrahydrofuran 6 Stunden bei Raumtemperatur gehalten. Man saugt bei 10°C ab, wäscht zuerst mit Tetrahydrofuran, dann mit Wasser und kristallisiert aus Methanol um. Man isoliert 4,7 g (62% d.Th.) 2-[p-(1-Chlorcyclopropylcarbonylamino)-phenyl]-3,4-diaza-bicyclo[4.1.0]hepten-(2)-on-(5), farblose Kristalle, Schmelzpunkt 221 bis 222°C.

Analyse für $C_{15}H_{14}ClN_3O_2$:

ber.: C 59,3 H 4,6 Cl 11,7 N 13,8%;
gef.: C 59,3 H 4,8 Cl 11,9 N 13,9%.

Beispiel 29

5,0 g (24,8 mmol) 2-(p-Aminophenyl)-3,4-diaza-bicyclo[4.1.0]hepten-(2)-on-(5) werden mit 5,2 g (30,0 mmol) 2,2-Dichlorcyclopropancarbonsäurechlorid und 100 ml absolutem Tetrahydrofuran 6 Stunden am Rückfluß gehalten. Man saugt bei 10°C ab, wäscht mit Wasser und kristallisiert aus Dimethylformamid/Wasser um. Man erhält 4,1 g (49% d.Th.) 2-[p-(2,2-Dichlorcyclopropylcarbonylamino)-phenyl]-3,4-diaza-bicyclo[4.1.0]hepten-(2)-on-(5), farblose Kristalle, Schmelzpunkt 264 bis 265°C.

Analyse für $C_{15}H_{13}Cl_2N_3O_2$:

ber.: C 53,3 H 3,9 Cl 21,8 N 12,4%;
gef.: C 52,9 H 3,9 Cl 20,8 N 12,5%.

Formulierungsbeispiele, die in üblicher Weise hergestellt werden:

1. Tabletten:

| | |
|---|---|
| Wirkstoff | 10 mg |
| Polyvinylpyrrolidon (mittl. M.G. 25 000) | 170 mg |
| Polyethylenglykol (mittl. M.G. 4 000) | 14 mg |
| Hydroxypropylmethylcellulose | 40 mg |
| Talkum | 4 mg |
| Magnesiumstearat | 2 mg |
| | 240 mg |

Der Wirkstoff wird mit Polyvinylpyrrolidon in 10%iger wäßriger Lösung befeuchtet, durch ein Sieb mit der lichten Maschenweite 1,0 mm getrieben und bei 50°C getrocknet. Dieses Granulat wird mit Polyethylenglykol (mittl. M.G. 4000), Hydroxypropylmethylcellulose, Talkum und Magnesiumstearat vermischt und zu Tabletten à 240 mg verpreßt.

2. Beispiel für Dragees:

| | |
|---|---|
| Wirkstoff | 10 mg |
| Lactose | 90 mg |
| Maisstärke | 60 mg |
| Polyvinylpyrrolidon | 6 mg |
| Magnesiumstearat | 1 mg |
| | 167 mg |

Die Mischung der Wirkstoffsubstanz mit Lactose und Maisstärke wird mit einer 8%igen wäßrigen Lösung des Polyvinylpyrrolidons durch ein Sieb 1,5 mm granuliert, bei 50°C getrocknet und nochmals durch ein Sieb 1,0 mm getrieben. Das so erhaltene Granulat wird mit Magnesiumstearat gemischt und zu Drageekernen verpreßt. Die erhaltenen Drageekerne werden in üblicher Weise mit einer Hülle überzogen, die im wesentlichen aus Zucker und Talkum besteht.

**Patentansprüche**

1. Verbindungen der Formel I,

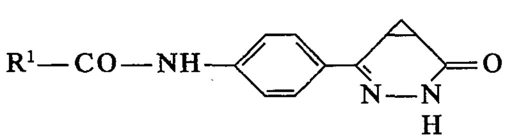

(I)

in der $R^1$ für ein Wasserstoffatom, einen gegebenenfalls durch 1 bis 3 Halogenatome substituierten Alkylrest mit 1 bis 4 C-Atomen, einen gegebenenfalls ein- bis dreifach durch Halogenatome und/oder Methylreste substituierten Cycloalkylrest mit 3 bis 5 C-Atomen im Ring oder einem Alkenylrest mit 2 bis 4 C-Atomen steht.

2. 2-(p-Aminophenyl)-3,4-diaza-bicyclo[4.1.0]hepten-(2)-on-(5).

3. 2-(p-Acetylaminophenyl)-3,4-diaza-bicyclo[4.1.0]hepten-(2)-on-(5).

4. 2-(p-Propionylaminophenyl)-3,4-diaza-bicyclo[4.1.0]hepten-(2)-on-(5).

5. 2-(p-Chloracetylaminophenyl)-3,4-diaza-bicyclo[4.1.0]hepten-(2)-on-(5).

6. 2-[p-(2-Chlorpropionylamino)-phenyl]-3,4-diaza-bicyclo[4.1.0]hepten-(2)-on-(5).

7. 2-(p-Cyclopropylcarbonylaminophenyl)-3,4-diaza-bicyclo[4.1.0]hepten-(2)-on-(5).

8. 2-(p-Cyclobutylcarbonylaminophenyl)-3,4-diaza-bicyclo[4.1.0]hepten-(2)-on-(5).

9. 2-(p-Acryloylaminophenyl)-3,4-diaza-bicyclo[4.1.0]hepten-(2)-on-(5).

10. Verfahren zur Herstellung von Verbindungen der Formel I

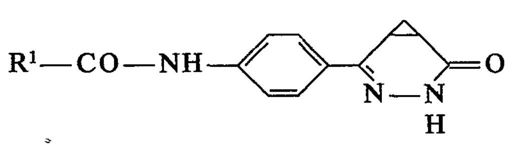

(I)

dadurch gekennzeichnet, daß man eine cis-2-Aroylcyclopropancarbonsäure der Formel III

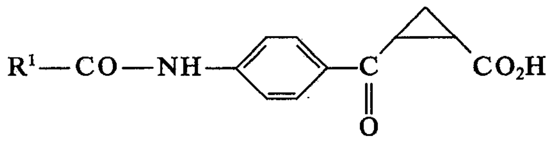

(III)

wobei $R^1$ jeweils die im Anspruch 1 angegebenen Bedeutungen hat, mit Hydrazin in an sich üblicher Weise cyclisiert.

11. Verfahren zur Herstellung von Verbindungen der Formel

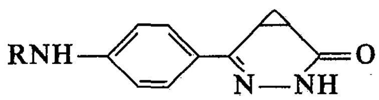

in der R ein Wasserstoffatom oder einen Acylrest der Formel $-COR^1$ bedeutet, worin $R^1$ für ein Wasserstoffatom, einen Alkylrest mit 1 bis 4 C-Atomen, der gegebenenfalls ein- bis dreifach durch Halogenatome substituiert ist, einen Cycloalkylrest mit 3 bis 5 C-Atomen im Ring, der gegebenenfalls ein- bis dreifach durch Halogenatome und/oder Methylreste substituiert ist, oder für einen Alkenylrest mit 2 bis 4 C-Atomen steht, dadurch gekennzeichnet, daß man eine Verbindung der Formel

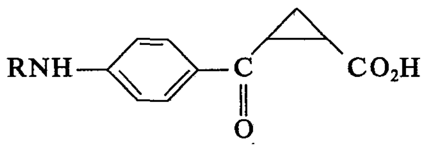

in der R die oben angegebenen Bedeutungen hat, mit Hydrazin in an sich üblicher Weise cyclisiert und gegebenenfalls eine erhaltene Aminoverbindung mit einem Acylierungsmittel der Formel $R^1COX$, in der $R^1$ die für Formel I angegebenen Bedeutungen hat und X für ein Chloratom, OH, einen niederen Alkoxyrest oder den Rest $OCOR^1$ steht, umsetzt oder gegebenenfalls eine erhaltene Acylaminoverbindung zur Aminoverbindung hydrolysiert.

12. Therapeutische Mittel, gekennzeichnet durch einen Gehalt an einer Verbindung aus Anspruch 1 als Wirkstoff neben üblichen Trägerstoffen und Verdünnungsmitteln.

13. Therapeutisches Mittel nach Anspruch 12, enthaltend 2-(p-Acetylaminophenyl)-3,4-diaza-bicyclo[4.1.0]hepten-(2)-on-(5) als Wirkstoff.

14. Therapeutisches Mittel nach Anspruch 12, enthaltend 2-(p-Propionylaminophenyl)-3,4-diaza-bicyclo[4.1.0]hepten-(2)-on-(5) als Wirkstoff.

15. Therapeutisches Mittel nach Anspruch 12, enthaltend 2-(p-Chloracetylaminophenyl)-3,4-diaza-bicyclo[4.1.0]hepten-(2)-on-(5) als Wirkstoff.

16. Therapeutisches Mittel nach Anspruch 12, enthaltend 2-[p-(2-Chlorpropionylamino)-phenyl]-3,4-diaza-bicyclo[4.1.0]hepten-(2)-on-(5) als Wirkstoff.

17. Therapeutisches Mittel nach Anspruch 12, enthaltend 2-(p-Cyclopropylcarbonylaminophenyl)-3,4-diaza-bicyclo[4.1.0]hepten-(2)-on-(5) als Wirkstoff.

18. Therapeutisches Mittel nach Anspruch 12, enthaltend 2-(p-Cyclobutylcarbonylaminophenyl)-3,4-diaza-bicyclo[4.1.0]hepten-(2)-on-(5) als Wirkstoff.

19. Therapeutisches Mittel nach Anspruch 12, enthaltend 2-(p-Acryloylaminophenyl)-3,4-diaza-bicyclo[4.1.0]hepten-(2)-on-(5) als Wirkstoff.

**Claims**

1. A compound of the formula I

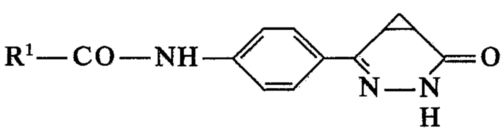

(I)

where $R^1$ is hydrogen, alkyl of 1 to 4 carbon atoms, which is unsubstituted or substituted by from 1 to 3 halogen atoms, cycloalkyl of 3 to 5 carbon atoms in the ring, which is unsubstituted or substituted by from 1 to 3 halogen atoms and/or methyl radicals, or alkenyl of 2 to 4 carbon atoms.

2. 2-(p-Aminophenyl)-3,4-diaza-bicyclo[4.1.0]hept-2-en-5-one.

3. 2-(p-Acetylaminophenyl)-3,4-diaza-bicyclo[4.1.0]hept-2-en-5-one.

4. 2-(p-Propionylaminophenyl)-3,4-diaza-bicyclo[4.1.0]hept-2-en-5-one.

5. 2-(p-Chloroacetylaminophenyl)-3,4-diaza-bicyclo[4.1.0]hept-2-en-5-one.

6. 2-[p-(2-Chloropropionylamino)-phenyl]-3,4-diaza-bicyclo[4.1.0]hept-2-en-5-one.

7. 2-(p-Cyclopropylcarbonylaminophenyl)-3,4-diaza-bicyclo[4.1.0]hept-2-en-5-one.

8. 2-(p-Cyclobutylcarbonylaminophenyl)-3,4-diaza-bicyclo[4.1.0]hept-2-en-5-one.

9. 2-(p-Acryloylaminophenyl)-3,4-diaza-bicyclo[4.1.0]hept-2-en-5-one.

10. A process for the preparation of a compound of the formula I

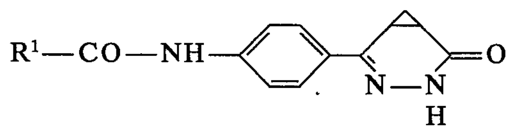

(I)

wherein a cis-2-aroylcyclopropanecarboxylic acid of the formula III

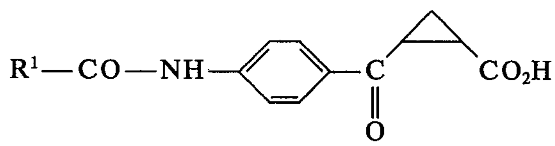

(III)

where $R^1$ has the meanings given in claim 1, is cyclized with hydrazine in a conventional manner.

11. A process for the preparation of a compound of the formula

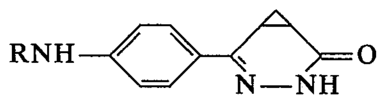

where R is hydrogen or acyl of the formula $-COR^1$, where $R^1$ is hydrogen, alkyl of 1 to 4 carbon atoms, which is unsubstituted or substituted by from 1 to 3 halogen atoms, cycloalkyl of 3 to 5 carbon atoms in the ring, which is unsubstituted or substituted by from 1 to 3 halogen atoms and/or methyl radicals, or alkenyl of 2 to 4 carbon atoms, wherein a compound of the formula

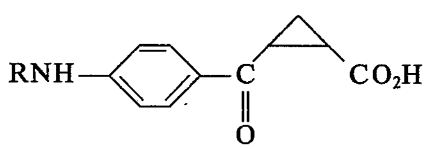

where R has the meanings given above, is cyclized with hydrazine in a conventional manner, and, if desired, an amino compound obtained is reacted with an acylating agent of the formula $R^1COX$, where $R^1$ has the meanings given for formula I and X is chlorine, OH, lower alkoxy or $OCOR^1$, or, if desired, an acylamino compound obtained is hydrolyzed to the amino compound.

12. A therapeutic agent which contains a compound as claimed in claim 1 as the active compound, in addition to conventional carriers and diluents.

13. A therapeutic agent as claimed in claim 12, which contains 2-(p-acetylaminophenyl)-3,4-diaza-bicyclo[4.1.0]hept-2-en-5-one as the active compound.

14. A therapeutic agent as claimed in claim 12, which contains 2-(p-propionylaminophenyl)-3,4-diaza-bicyclo[4.1.0]hept-2-en-5-one as the active compound.

15. A therapeutic agent as claimed in claim 12, which contains 2-(p-chloroacetylaminophenyl)-3,4-diaza-bicyclo[4.1.0]hept-2-en-5-one as the active compound.

16. A therapeutic agent as claimed in claim 12, which contains 2-[p-(2-chloropropionylamino)-phenyl]-3,4-diaza-bicyclo[4.1.0]hept-2-en-5-one as the active compound.

17. A therapeutic agent as claimed in claim 12, which contains 2-(p-cyclopropylcarbonylamino-phenyl)-3,4-diaza-bicyclo[4.1.0]hept-2-en-5-one as the active compound.

18. A therapeutic agent as claimed in claim 12, which contains 2-(p-cyclobutylcarbonylamino-phenyl)-3,4-diaza-bicyclo[4.1.0]hept-2-en-5-one as the active compound.

19. A therapeutic agent as claimed in claim 12, which contains 2-(p-acryloylaminophenyl)-3,4-diaza-bicyclo[4.1.0]hept-2-en-5-one as the active compound.

**Revendications**

1. Composés de formule I

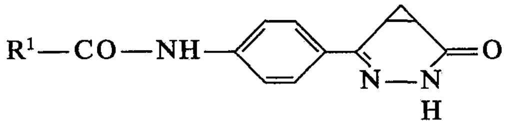

(I)

dans laquelle R[1] représente un atome d'hydrogène, un reste alkyle de 1 à 4 atomes C, éventuellement substitué par 1 à 3 atomes d'halogène, un reste cycloalkyle de 3 à 5 atomes C dans le noyau, éventuellement substitué une à trois fois par des atomes d'halogène et/ou des restes méthyle, ou un reste alcényle de 2 à 4 atomes C.

2. 2-(p-aminophényl)-3,4-diaza-bicyclo[4.1.0]hepten-(2)-one-(5).
3. 2-(p-acétylaminophényl)-3,4-diaza-bicyclo[4.1.0]hepten-(2)-one-(5).
4. 2-(p-propionylaminophényl)-3,4-diaza-bicyclo[4.1.0]hepten-(2)-one-(5).
5. 2-(p-chloracétylaminophényl)-3,4-diaza-bicyclo[4.1.0]hepten-(2)-one-(5).
6. 2-[p-(2-chloropropionylamino)-phényl]-3,4-diaza-bicyclo[4.1.0]hepten-(2)-one-(5).
7. 2-(p-cyclopropylcarbonylaminophényl)-3,4-diaza-bicyclo[4.1.0]hepten-(2)-one-(5).
8. 2-(p-cyclobutylcarbonylaminophényl)-3,4-diaza-bicyclo[4.1.0]hepten-(2)-on-(5).
9. 2-(p-acryloylaminophényl)-3,4-diaza-bicyclo[4.1.0]hepten-(2)-one-(5).
10. Procédé de préparation de composés de formule I

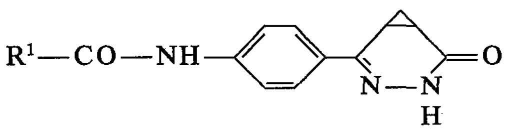

(I)

caractérisé par le fait qu'on cyclise, de manière usuelle, avec de l'hydrazine, un acide cis-2-aroylcyclopropanecarboxylique de formule III

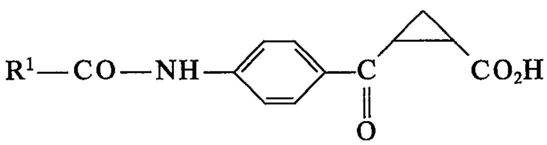

(III)

R[1] ayant les significations données dans la revendication 1.

11. Procédé de préparation de composés de formule

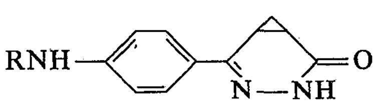

dans laquelle R représente un atome d'hydrogène ou un reste acyle de formule $-COR^1$, où R[1] représente un atome d'hydrogène, un reste alkyle de 1 à 4 atomes C, qui est éventuellement substitué une à trois fois par des atomes d'halogène, un reste cycloalkyle de 3 à 5 atomes C dans le noyau, qui est éventuellement substitué une à trois fois par des atomes d'halogène et/ou restes méthyle, ou un reste alcényle de 2 à 4 atomes C, caractérisé par le fait qu'on cyclise, de manière habituelle en soi, un composé de formule

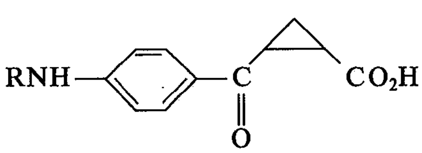

dans laquelle R a les significations données plus haut, avec de l'hydrazine, et qu'on fait réagier éventuellement un composé amino obtenu, avec un agent d'acylation de formule $R^1COX$, dans laquelle R[1] a les significations donées pour la formule I, et X représente un atome de chlore, OH, un reste alcoxy inférieur ou le reste $OCOR^1$, ou on hydrolyse éventuellement en composé amino un composé acylamino obtenu.

12. Agent thérapeutique, caractérisé par le fait qu'il contient, comme principe actif, un

composé selon la revendication 1, outre des véhicules et diluants usuels.

13. Agent thérapeutique selon la revendication 12, contenant comme principe actif 2-(p-acétylaminophényl)-3,4-diaza-bicyclo[4.1.0]hepten-(2)-one-(5).

14. Agent thérapeutique selon la revendication 12, contenant comme principe actif 2-(p-propionylaminophényl)-3,4-diaza-bicyclo[4.1.0]hepten-(2)-one-(5).

15. Agent thérapeutique selon la revendication 12, contenant comme principe actif 2-(p-chloracétylaminophényl)-3,4-diaza-bicyclo[4.1.0]hepten-(2)-one-(5).

16. Agent thérapeutique selon la revendication 12, contenant comme principe actif 2-[p-(2-chloropropionylamino)-phényl]-3,4-diaza-bicyclo[4.1.0]hepten-(2)-one-(5).

17. Agent thérapeutique selon la revendication 12, contenant comme principe actif 2-(p-cyclopropylcarbonylaminophényl)-3,4-diaza-bicyclo[4.1.0]hepten-(2)-one-(5).

18. Agent thérapeutique selon la revendication 12, contenant comme principe actif 2-(p-cyclobutylcarbonylaminophényl)-3,4-diaza-bicyclo[4.1.0]hepten-(2)-one-(5).

19. Agent thérapeutique selon la revendication 12, contenant comme principe actif 2-(p-acryloylaminophényl)-3,4-diaza-bicyclo[4.1.0]hepten-(2)-one-(5).